# EUROPÄISCHE PATENTSCHRIFT

(11) **EP 0 635 482 B1**
(45) Veröffentlichungstag und Bekanntmachung des Hinweises auf die Patenterteilung: **14.10.1998**
(21) Anmeldenummer: 94110879.7
(22) Anmeldetag: 13.07.1994
(51) Int. Cl.: C07C 201/12, C07C 205/12

(54) **Verfahren zur Herstellung von mehrfach fluorierten Nitrobenzolen**
Process for the preparation of polyfluoronitrobenzenes
Procédé pour la préparation de polyfluoronitrobenzènes

(30) Priorität: 21.07.1993 DE 4324365
(43) Veröffentlichungstag der Anmeldung: 25.01.1995
(73) Patentinhaber: Clariant GmbH, 65929 Frankfurt am Main (DE)
(72) Erfinder: Schach, Dr. Thomas, D-64579 Gernsheim (DE); Papenfuhs, Dr. Theodor, D-60433 Frankfurt am Main (DE)

(56) Entgegenhaltungen:
- EP-A- 0 270 965
- EP-A- 0 354 444
- EP-A- 0 523 671
- WO-A-87/04149
- US-A- 4 694 104

## Beschreibung

Gegenstand der vorliegenden Erfindung ist ein verbessertes Verfahren zur Herstellung von mehrfach fluorierten Nitrobenzolen durch Umsetzung der entsprechenden Chlornitrobenzole mit Alkalimetallfluoriden in Gegenwart eines neuen Katalysatorsystems.
Der Halogenaustausch, bevorzugt der von aktivierten Chlornitrobenzolen oder Bromnitrobenzolen ist eine übliche Methode, Fluorsubstituenten in ein aromatisches System einzuführen. Im allgemeinen wird die Reaktion in Gegenwart von aprotisch dipolaren Lösungsmitteln und Alkalimetallfluoriden als Fluoridquelle durchgeführt (US-A-3 064 058). Markante Nachteile dieser Verfahren sind die hohen Reaktionstemperaturen, mäßige Produktausbeuten und lange Reaktionszeiten.

Als Alternative können herkömmliche Phasentransferkatalysatoren verwendet werden, durch die sich einige der oben beschriebenen Nachteile verbessern lassen. Andere Probleme, wie beispielsweise eine schlechte Rührbarkeit der Reaktionssuspension bei lösungsmittelfreien Verfahren bleiben weiter bestehen. Bislang wurden als Phasentransferkatalysatoren quartäre Alkylammonium- oder Alkylphosphoniumsalze (US-A-4,287,374), Pyridiniumsalze (WO-A-87/04149) oder Kronenether verwendet, die zum Teil nur geringe Reaktivitäten zeigen oder unter den benötigten Reaktionstemperaturen nur mäßig stabil sind.

Aus US-A-4694104 gehen quaternäre Polyoxyalkylen-Salze als Phasentransferkatalysatoren für eine flüssig/flüssig-Phasen-Cyclopropanierung hervor.

Mehrfache Chlor-Fluor-Austauschreaktionen ohne Lösungsmittel waren bislang nur bedingt möglich. Der hohe Salzgehalt der Reaktionssuspension führte in der Regel zu nicht rührbaren Systemen, die selbst unter günstigsten Bedingungen nur zu geringen Umsätzen und Ausbeuten führen. Bislang gelang ein doppelter Cl/F-Austausch nur in Gegenwart geeigneter Lösungmittel, wie beispielsweise Sulfolan oder Dimethylsulfoxid (US-A-4,164,517, DE-A-3 642 332).

In Anbetracht dieser Einschränkungen und Nachteile bestand ein großes Bedürfnis nach einem verbesserten Verfahren, durch das die den bekannten Verfahren innewohnenden Nachteile vermieden und gute bis sehr gute Ausbeuten, niedrigere Reaktionstemperaturen und verkürzte Reaktionszeiten ermöglicht und geringere Mengen an polymeren Zersetzungsprodukten erhalten werden. Insbesondere der Bewältigung von Rührproblemen und Aufarbeitungsproblemen in lösungsmittelfreien Verfahren und in Verfahren mit nur sehr geringen Lösungsmittelmengen wurde eine besondere Bedeutung beigemessen.

Es wurde gefunden, daß man Nitrobenzole in vorteilhafter Weise mehrfach fluorieren kann, indem man die entsprechenden Chlornitrobenzole mit Alkalimetallfluoriden in Gegenwart einer quartären Ammoniumverbindung, die mindestens einen Alkoxypolyoxyalkyl-Rest enthält, umsetzt.

Gegenstand der vorliegenden Erfindung ist ein Verfahren zur Herstellung mehrfach fluorierter Nitrobenzole durch Umsetzung einer Verbindung der Formel (4) 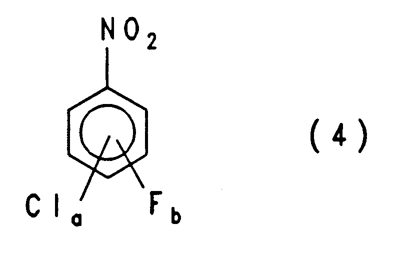 worin
a eine Zahl von 2 bis 4 und
b eine Zahl von 0 bis 2 ist,
mit einem Alkalimetallfluorid bei einer Reaktionstemperatur von 80 bis 220°C in Gegenwart eines Katalysators, dadurch gekennzeichnet, daß der Katalysator im wesentlichen aus
a) einer oder mehreren quartären Ammoniumverbindung(en) der Formel (1) 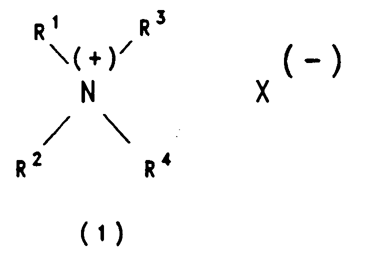 worin
   - R¹, R² und R³: gleich oder verschieden sind und
   einen linearen oder verzweigten Alkoxypolyoxyalkyl-Rest der Formel -(CₘH₂ₘO)ₚR⁵ bedeuten, worin R⁵ für Wasserstoff oder einen linearen oder verzweigten Alkylrest mit 1 bis 16, vorzugsweise 1 bis 8, Kohlenstoffatomen steht, m eine ganze Zahl von 1 bis 10, vorzugsweise 1 bis 5, und p eine Zahl von 1 bis 15, vorzugsweise 2 bis 10, bedeuten; oder
   einen linearen oder verzweigten Alkylrest mit 1 bis 30, vorzugsweise 1 bis 18, Kohlenstoffatomen; oder einen unsubstituierten Phenyl-oder Naphthylrest; oder einen substituierten Phenyl- oder Naphthylrest, wobei die Substituenten die Bedeutung Halogen, C₁-C₄-Alkyl, C₁-C₄-Alkoxy, Nitro oder Cyano haben;
   - R⁴: einen linearen oder verzweigten Alkoxypolyoxyalkyl-Rest der Formel -(CₘH₂ₘO)ₚR⁵ bedeutet; und
   - X^{⊖}: ein anorganisches Anion, vorzugsweise Fluorid, Chlorid, Bromid, SO₄²⁻/2 oder Hydrogensulfat ist;
   oder aus einer Mischung der Komponente a) und
b) einem oder mehreren quartären Ammoniumsalz(en) oder Phosphoniumsalz(en) der Formel (2) 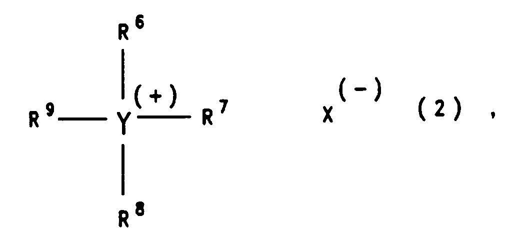 worin
   - R⁶, R⁷, R⁸ und R⁹: gleich oder verschieden sind und
   einen linearen oder verzweigten Alkylrest mit 1 bis 22, vorzugsweise 1 bis 16, Kohlenstoffatomen; oder einen unsubstituierten oder substituierten Arylrest oder einen C₁-C₄-Alkyl-arylrest, wobei Aryl die Bedeutung Phenyl oder Naphthyl hat und die besagten Substituenten Halogen, C₁-C₄-Alkyl, C₁-C₄-Alkoxy, Nitro oder Cyano bedeuten; und
   - Y: die Bedeutung N oder P hat;
   oder aus einer Mischung der Komponente a) und
c) einem oder mehreren Polyether(n) der Formel (3)

   R¹⁰-(O-CₓH₂ₓ)ᵣ-OR¹¹ (3),

   worin
   - R¹⁰ und R¹¹: gleich oder verschieden sind und Wasserstoff oder einen linearen oder verzweigten Alkylrest mit 1 bis 16, vorzugsweise 1 bis 8, Kohlenstoffatomen bedeuten,
   - x: eine ganze Zahl von 2 bis 6, vorzugsweise 2 bis 3, und
   - r: eine Zahl von 0 bis 20, vorzugsweise 4 bis 14, ist;
   oder einem Kronenether;
   oder aus einer Mischung der Komponenten a), b) und c) besteht.

Der Katalysator besteht vorzugsweise nur aus Komponente a), es kann jedoch zweckmäßig sein, eine Mischung aus den Komponenten a) und b) oder aus den Komponenten a) und c) oder aus den Komponenten a), b) und c) einzusetzen.

Die Mischungsverhältnisse der Komponenten a) und b), a) und c) sowie a), b) und c) können in einem weiten Bereich schwanken, mit der Maßgabe, daß die Komponente a) mindestens 5 Gew.-%, vorzugsweise 20 bis 80 Gew.-%, des gesamten Katalysators ausmacht.

In dem in der Verbindung der Formel (1) enthaltenen linearen oder verzweigten Alkoxypolyoxyalkyl-Rest der Formel -(CₘH₂ₘO)ₚR⁵ können gleiche oder unterschiedliche Alkoxy-Einheiten miteinander verknüpft sein. Die Anzahl der in der Verbindung der Formel (1) enthaltenen linearen oder verzweigten Alkoxypolyoxyalkyl-Reste beträgt vorzugsweise 1 oder 2. Besonders bevorzugte Verbindungen der Formel (1) im Sinne der vorliegenden Erfindung sind Dimethyl-di(ethoxypolyoxypropyl)-ammoniumchlorid, Dimethyl-di(ethoxypolyoxypropylmethylether)-ammoniumchlorid, Dimethyl-(ethoxypolyoxypropyl)-(ethoxypolyoxypropylmethylether)-ammoniumchlorid, Dimethyl-di(ethoxypolyoxyethyl)-ammoniumchlorid, Dimethyl-di(ethoxypolyoxyethylmethylether)-ammoniumchlorid, Dimethyl-(ethoxypolyoxyethyl)-(ethoxypolyoxyethylmethylether)-ammoniumchlorid, jeweils mit einer mittleren Kettenlänge p von 3, weiterhin Trimethyl-(ethoxypolyoxypropyl)-ammoniumchlorid und Trimethyl-(ethoxypolyoxypropylmethylether)-ammoniumchlorid, jeweils mit einer mittleren Kettenlänge p von 8, oder ein Gemisch der genannten Verbindungen.

Die beschriebenen Verbindungen der Formel (1) lassen sich auf bekannte Weise (US-A-3,123,641; US-A-3,141,905) aus den entsprechenden Ethanolaminen herstellen, die nach Umsetzung mit Alkylenoxiden und anschließender Quaternisierung mit oder ohne gleichzeitiger Veretherung in guten Ausbeuten die gewünschten Verbindungen liefern.

Bevorzugte Verbindungen der Formel (2) im Sinne der vorliegenden Erfindung sind Octadecyltrimethylammoniumchlorid, Distearyldimethylammoniumchlorid, Tetramethylammoniumchlorid, Tetramethylammoniumbromid, Hexadecyltrimethylammoniumchlorid, Benzyltrimethylammoniumchlorid, Hexadecyltributylphosphoniumbromid, Stearyltributylphosphoniumbromid, Tetrabutylphosphoniumchlorid, Tetrabutylphosphoniumbromid und Tetraoctylphosphoniumbromid.

Bevorzugte Polyether der Formel (3) im Sinne der vorliegenden Erfindung besitzen eine mittlere Molmasse zwischen 300 und 800. Besonders bevorzugt ist ein Gemisch von Polyethylenglykoldimethylethern der Kettenlängen r von 6 bis 17 und einer mittleren Molmasse von 500. Anstelle von oder in Kombination mit Polyethern der Formel (3) können auch übliche Kronenether, beispielsweise 18-Krone-6, eingesetzt werden.

Als Ausgangsverbindungen der Formel (4) für das erfindungsgemäße Verfahren kommen in Betracht:
Dichlornitrobenzole, wie beispielsweise 2,4-Dichlornitrobenzol, 2,4-Dichlor-3-fluornitrobenzol und 2,4-Dichlor-5-fluornitrobenzol; Trichlornitrobenzole, wie beispielsweise 2,4,5-Trichlornitrobenzol, 2,3,4-Trichlornitrobenzol, 2,3,4-Trichlor-5-fluornitrobenzol und 2,4,5-Trichlor-3-fluornitrobenzol.

Durch das erfindungsgemäße Verfahren können in den genannten Ausgangsverbindungen zwei oder drei, vorzugsweise zwei, Chloratome durch Fluoratome ausgetauscht werden, so daß als Endprodukte beispielsweise erhalten werden:
Difluornitrobenzole, wie beispielsweise 2,4-Difluornitrobenzol, 5-Chlor-2,4-difluornitrobenzol und 3-Chlor-2,4-difluornitrobenzol; Trifluornitrobenzole, wie beispielsweise 2,3,4-Trifluornitrobenzol, 2,4,5-Trifluornitrobenzol, 3-Chlor-2,4,5-trifluornitrobenzol und 5-Chlor-2,3,4-trifluornitrobenzol.

Als Alkalimetallfluoride werden vorzugsweise Kaliumfluorid, Rubidiumfluorid oder Cäsiumfluorid oder Kombinationen aus diesen verwendet, insbesondere Kaliumfluorid. Vorteilhaft bei dem erfindungsgemäßen Verfahren ist, daß Alkalimetallfluoride eingesetzt werden können, deren Wassergehalt bis zu 3 % betragen kann. Dadurch ist es beispielsweise möglich, technisches Kaliumfluorid ohne Vorbehandlung zu verwenden.

Bei dem erfindungsgemäßen Verfahren wird der Katalysator zweckmäßigerweise in Mengen von 1 bis 35 Gew.-%, vorzugsweise von 5 bis 15 Gew.-%, bezogen auf die aromatische Ausgangsverbindung, eingesetzt. Das molare Verhältnis von Katalysator zur Ausgangsverbindung ist dabei gleich oder kleiner als 1:10, bevorzugt 1:15 bis 1:50.
Was das Mengenverhältnis des Alkalimetallfluorids zur Ausgangsverbindung anbelangt, so werden zweckmäßigerweise 60 bis 200 Mol-%, vorzugsweise 100 bis 140 Mol-%, bezogen auf jedes auszutauschende Chloratom, Alkalimetallfluorid eingesetzt.

Zwei- oder dreifache Chlor-Fluor-Austauschreaktionen zur Herstellung von Fluornitrobenzolen waren bislang nur in Gegenwart von Lösungsmitteln möglich. Der hohe Salzgehalt der Reaktionssuspension führte in der Regel zu nicht rührbaren Systemen, die selbst unter günstigsten Bedingungen nur zu geringen Umsätzen und Ausbeuten führten. Bei dem erfindungsgemäßen Verfahren kommt es nunmehr selbst bei sehr hohen Salzgehalten in der Reaktionssuspension zu keinen Rührproblemen, so daß auch zwei- oder dreifache Austausch-Reaktionen meist problemlos ohne Lösungsmittel durchgeführt werden. Die gleichzeitig deutlich niedrigeren Reaktionstemperaturen im Vergleich zum Stand der Technik führen zusammen mit der guten Rührbarkeit der Reaktionssuspension letztendlich zu einer deutlichen Steigerung der Ausbeute und einer Verminderung von Nebenreaktionen.

Während bislang Temperaturen von 200°C bis über 300°C für Chlor-Fluoraustauschreaktionen erforderlich waren, liegen die Reaktionstemperaturen des erfindungsgemäßen Verfahrens bei 80 bis 220°C, vorzugsweise bei 90 bis 180°C, insbesondere bei 120 bis 170°C.

Das erfindungsgemäße Verfahren kann in Gegenwart oder Abwesenheit von Lösungsmitteln durchgeführt werden. Werden Lösungsmittel verwendet, so sind sowohl aprotische und dipolar aprotische als auch protische Lösungsmittel geeignet. Geeignete dipolar aprotische Lösungsmittel sind beispielsweise Dimethylsulfoxid, Dimethylsulfon, Sulfolan, Dimethylformamid, Dimethylacetamid, 1,3-Dimethylimidazolin-2-on, Acetonitril und Benzonitril. Geeignete aprotische Lösungsmittel ohne ausgeprägten dipolaren Charakter sind beispielsweise Benzol, Toluol, Xylol, Chlortoluole, Chlorbenzol und Dichlorbenzole. Die Verwendung von protischen Lösungsmitteln, wie beispielsweise Alkoholen, ist ebenfalls möglich. Als protische Lösungsmittel werden Methanol, Ethanol, Propanol, Butanol, i-Propanol oder Polyalkylenglykole mit Ethylen-, Propylen- oder Butyleneinheiten verwendet.

Das aprotische oder dipolar aprotische Lösungsmittel kann in beliebigen Mengen verwendet werden, bevorzugt werden allerdings geringe Mengen im Bereich von 5 bis 30 Gew.-%, bezogen auf den eingesetzten Aromaten. Bei der Verwendung von protischen Lösungsmitteln liegen die eingesetzten Mengen im Bereich von 0,1 bis 5 Gew.-%, bevorzugt 0,1 bis 2 Gew.-%, bezogen auf den eingesetzten Aromaten.

Der erfindungsgemäße Katalysator kann sowohl bei Atmosphärendruck als auch bei Überdruck- oder Unterdruck verwendet werden. Diese Eigenschaften wird beispielsweise genutzt, indem geringe Mengen eines leichtsiedenden aprotischen Lösungsmittels, das mit Wasser ein Azeotrop bildet, wie beispielsweise Benzol, Xylol, Mesitylen oder Toluol, vor Beginn der Reaktion in die Reaktionssuspension gegeben werden. Anschließend wird ein Teil des Lösungsmittels durch Anlegen eines Unterdrucks zusammen mit Wasser aus der Reaktionssuspension wieder entfernt. Durch diese Verfahrensweise lassen sich die Reaktionsgeschwindigkeit und die Ausbeute steigern und die Bildung von Nebenprodukten minimieren.

Das erfindungsgemäße Verfahren kann in Anwesenheit oder Abwesenheit von Luftsauerstoff durchgeführt werden, bevorzugt wird das Arbeiten unter Schutzgas, wie beispielsweise Argon oder Stickstoff. Bei dem erfindungsgemäßen Verfahren ist zu gewährleisten, daß während der gesamten Reaktion das Reaktionsgemisch gut durchmischt wird.

Fluornitrobenzole spielen eine bedeutende Rolle als Zwischenprodukte im Bereich des Pflanzenschutzes und als Synthesebausteine für Pharmazeutika und Farbstoffe.

Die folgenden Beispiele dienen zur Erläuterung des erfindungsgemäßen Verfahrens, ohne sich darauf zu beschränken. Unter "Polyethylenglykoldimethylether 500" wird besagter Polyether mit einer mittleren Molmasse von etwa 500 verstanden.

Das in den Beispielen verwendete Trimethyl-(ethoxypolyoxypropyl)-ammoniumchlorid hat eine mittlere Kettenlänge p von 8 und wurde als 84 bis 89 gew-%iges Produkt eingesetzt. In diesem Produkt sind noch 10 bis 13 Gew.-% freies Polypropylenglykol und bis zu 2 Gew.-% Wasser enthalten.

Das eingesetzte Dimethyl-di(ethoxypolyoxypropyl)-ammoniumchlorid hat eine mittlere Kettenlänge p von 3 und ist ein 90 bis 95 gew.-%iges Produkt, das noch 5 bis 10 Gew.-% Polypropylenglykol und etwa 0,2 Gew.-% Wasser enthält.

Wurden die beiden Katalysatoren als veretherte Verbindungen verwendet, so lagen die Polypropylenglykole ebenfalls in veretherter Form vor. Der Veretherungsgrad lag im Falle von Dimethoxy-di(ethoxypolyoxypropylmethylether)-ammoniumchlorid bei 86 %.

Der zeitliche Reaktionsverlauf wurde durch gaschromatographische Analyse (GC) verfolgt und die jeweils in der Reaktionsmischung vorhandene Menge des gewünschten Produktes in Form von GC-Flächenprozenten angegeben.

### Beispiel 1: 2,4-Difluornitrobenzol

In einen 2,5-Liter Planschliffkolben mit Destillationsbrücke und Ankerrührer wurden bei 100°C in die Schmelze von 768 g (4 mol) 2,4-Dichlornitrobenzol 511 g (8,8 mol) Kaliumfluorid, 89,9 g (0,17 mol) Dimethyl-di(ethoxypolyoxypropyl)-ammoniumchlorid und 44,5 g (0,09 mol) Polyethylenglykoldimethylether 500 eingetragen. Anschließend wurden 50 g (0,47 mol) Xylol zugegeben und die Reaktionssuspension durch Anlegen eines Vakuums von 20 mbar und Erhitzen auf 130°C azeotrop getrocknet. Nachdem das Xylol abdestilliert war, wurde die Destillationsbrücke gegen einen Rückflußkühler ausgetauscht, die Reaktionssuspension auf 140°C erhitzt und 21 Stunden bei dieser Temperatur gerührt. Anschließend wurde die Reaktionssuspension auf 70°C abgekühlt und abgesaugt (70°C). Die abgetrennten Salze wurden zweimal mit insgesamt 180 g Xylol nachgewaschen und die vereinigten organischen Phasen fraktioniert. Es wurden 535 g (84 % d.Th.) 2,4-Difluornitrobenzol isoliert. Entstandene Menge an 2,4-Difluornitrobenzol nach GC-Analyse: nach 6 Stunden 57 % GC Flächen-%, nach 21 Stunden 86 % GC Flächen-%.

### Beispiel 2: 2,4-Difluornitrobenzol

In einen 2,5-Liter Planschliffkolben mit Destillationsbrücke und Ankerrührer wurden bei 100°C in die Schmelze von 768 g (4 mol) 2,4-Dichlornitrobenzol 511 g (8,8 mol) Kaliumfluorid und 40 g (0,07 mol) Dimethyl-di(ethoxypolyoxypropyl)-ammoniumchlorid, 30,0 g (0,06 mol) Polyethylenglykoldimethylether 500 und 11,9 g (0,035 mol) Tetrabutylphosphoniumbromid eingetragen. Anschließend wurden 80 g (0,75 mol) Xylol zugegeben und die Reaktionssuspension durch Anlegen eines Vakuums von 30 mbar und Erhitzen auf 130°C azeotrop getrocknet. Nachdem das Xylol abdestilliert war, wurde die Destillationsbrücke gegen einen Rückflußkühler ausgetauscht, die Reaktionssuspension auf 140°C erhitzt und 21 Stunden bei dieser Temperatur gerührt. Anschließend wurde die Reaktionssuspension auf 70°C abgekühlt und abgesaugt (70°C). Die abgetrennten Salze wurden zweimal mit insgesamt 180 g Xylol nachgewaschen und die vereinigten organischen Phasen fraktioniert. Es wurden 553 g (87 % d.Th.) 2,4-Difluornitrobenzol isoliert. Entstandene Menge an 2,4-Difluornitrobenzol nach GC-Analyse: nach 5 Stunden 23 GC Flächen-%, nach 21 Stunden 89 GC Flächen-%.

### Beispiel 3: 2,4-Difluornitrobenzol

In einen 500 ml Planschliffkolben mit Destillationsbrücke und Impellerrührer wurden bei 100°C in die Schmelze von 120 g (0,62 mol) 2,4-Dichlornitrobenzol 72,0 g (1,24 mol) Kaliumfluorid, 14,0 g (0,02 mol) Dimethyl-di(ethoxypolyoxypropyl)-ammoniumchlorid und 7,0 g (0,013 mol) Polyethylenglykoldimethylether 500 eingetragen. Die Temperatur wurde auf 120°C erhöht und die Reaktionssuspension 28 Stunden bei dieser Temperatur gerührt. Entstandene Menge an 2,4-Difluornitrobenzol: nach 6 Stunden 11 GC-Flächen-%, nach 28 Stunden 56 GC-Flächen-%.

### Beispiel 4: 2,3,4-Trifluornitrobenzol

In einen 2,5-Liter Planschliffkolben mit Destillationsbrücke und Ankerrührer wurden bei 110°C in die Schmelze von 840 g (4 mol) 2,4-Dichlor-3-fluornitrobenzol 581 g (10,0 mol) Kaliumfluorid, 71,1 g (0,1 mol) Trimethyl-(ethoxypolyoxypropyl)-ammoniumchlorid, 20,0 g (0,04 mol) Polyethylenglykoldimethylether 500 und 13,6 g (0,04 mol) Tetrabutylphosphoniumbromid eingetragen. Anschließend wurden 80 g (0,75 mol) Xylol zugegeben und die Reaktionssuspension durch Anlegen eines Vakuums von 30 mbar und Erhitzen auf 120°C azeotrop getrocknet. Nachdem das Xylol abdestilliert war, wurde die Destillationsbrücke gegen einen Rückflußkühler ausgetauscht, die Reaktionssuspension auf 130°C erhitzt und 21 Stunden unter guter Rührung bei dieser Temperatur gerührt. Anschließend wurde die Reaktionssuspension auf 25°C abgekühlt und abgesaugt (25°C). Die abgetrennten Salze wurden zweimal mit insgesamt 180 g Xylol nachgewaschen und die vereinigten organischen Phasen fraktioniert. Es wurden 506 g (72 % d.Th.) 2,3,4-Trifluornitrobenzol isoliert. Entstandene Menge an 2,3,4-Trifluornitrobenzol nach GC-Analyse: nach 5 Stunden 15 GC-Flächen-%, nach 21 Stunden 74-GC Flächen-%.

### Beispiel 5: 5-Chlor-2,4-difluornitrobenzol

In einen 2,5-Liter Planschliffkolben mit Destillationsbrücke und Ankerrührer wurden bei 100°C in die Schmelze von 905 g (4 mol) 2,4,5-Trichlornitrobenzol 523 g (9,0 mol) Kaliumfluorid und 85,3 g (0,12 mol) Trimethyl-(ethoxypolyoxypropyl)-ammoniumchlorid und 19,3 g (0,06 mol) Tetrabutylphosphoniumbromid eingetragen. Anschließend wurden 80 g (0,75 mol) Xylol zugegeben und die Reaktionssuspension durch Anlegen eines Vakuums von 40 mbar und Erhitzen auf 120°C azeotrop getrocknet. Nachdem das Xylol abdestilliert war, wurde die Destillationsbrücke gegen einen Rückflußkühler ausgetauscht, die Reaktionssuspension auf 130°C erhitzt und 21 Stunden bei dieser Temperatur gerührt. Anschließend wurde die Reaktionssuspension auf 25°C abgekühlt und abgesaugt (25°C). Die abgetrennten Salze wurden zweimal mit insgesamt 180 g Xylol nachgewaschen und die vereinigten organischen Phasen fraktioniert. Es wurden 619 g (80 % d.Th.) 5-Chlor-2,4-difluornitrobenzol isoliert. Entstandene Menge an 5-Chlor-2,4-difluornitrobenzol nach GC-Analyse: nach 5 Stunden 38 GC-Flächen-%, nach 21 Stunden 89-GC-Flächen-%.

### Beispiel 6: 2,4,5-Trifluornitrobenzol

In einen 2,5-Liter Planschliffkolben mit Destillationsbrücke und Ankerrührer wurden bei 110°C in die Schmelze von 840 g (4 mol) 2,4-Dichlor-5-fluornitrobenzol 581 g (10,0 mol) Kaliumfluorid, 71,1 g (0,1 mol) Trimethyl-(ethoxypolyoxypropyl)-ammoniumchlorid, 20,0 g (0,04 mol) Polyethylenglykoldimethylether 500 und 13,6 g (0,04 mol) Tetrabutylphosphoniumbromid eingetragen. Anschließend wurden 80 g (0,75 mol) Xylol zugegeben und die Reaktionssuspension durch Anlegen eines Vakuums von 30 mbar und Erhitzen auf 120°C azeotrop getrocknet. Nachdem das Xylol abdestilliert war, wurde die Destillationsbrücke gegen einen Rückflußkühler ausgetauscht, die Reaktionssuspension auf 130°C erhitzt und 21 Stunden bei dieser Temperatur gerührt. Anschließend wurde die Reaktionssuspension auf 25°C abgekühlt und abgesaugt (25°C). Die abgetrennten Salze wurden zweimal mit insgesamt 180 g Xylol nachgewaschen und die vereinigten organischen Phasen fraktioniert. Es wurden 506 g (72 % d.Th.) 2,4,5-Trifluornitrobenzol isoliert. Entstandene Menge an 2,4,5-Trifluornitrobenzol nach GC-Analyse: nach 5 Stunden 15 GC-Flächen-%, nach 21 Stunden 74 GC-Flächen-%.

### Beispiel 7: 2,3,4-Trifluornitrobenzol

In einen 2,5-Liter Planschliffkolben mit Destillationsbrücke und Ankerrührer wurden bei 110°C in die Schmelze von 840 g (4 mol) 2,4-Dichlor-3-fluornitrobenzol 488 g (8,4 mol) Kaliumfluorid, 40,0 g (0,06 mol) Trimethyl-(ethoxypolyoxypropylmethylether)-ammoniumchlorid, 20,0 g (0,04 mol) Polyethylenglykoldimethylether 500 und 20,4 g (0,06 mol) Tetrabutylphosphoniumbromid eingetragen. Anschließend wurden 60 g (0,57 mol) Xylol zugegeben und die Reaktionssuspension durch Anlegen eines Vakuums von 30 mbar und Erhitzen auf 130°C azeotrop getrocknet. Nachdem das Xylol abdestilliert war, wurde die Destillationsbrücke gegen einen Rückflußkühler ausgetauscht, die Reaktionssuspension auf 150°C erhitzt und 21 Stunden bei dieser Temperatur gerührt. Anschließend wurde die Reaktionssuspension auf 25°C abgekühlt und abgesaugt (25°C). Die abgetrennten Salze wurden zweimal mit insgesamt 400 g Xylol nachgewaschen und die vereinigten organischen Phasen fraktioniert. Es wurden 516,8 g (73 % d.Th.) 2,3,4-Trifluornitrobenzol isoliert. Entstandene Menge an 2,3,4-Trifluornitrobenzol nach GC-Analyse: nach 5 Stunden 14 GC-Flächen-%, nach 21 Stunden 75-GC-Flächen-%.

### Beispiel 8: 2,3,4-Trifluornitrobenzol

In einen 1,5-Liter Planschliffkolben mit Destillationsbrücke und Ankerrührer wurden bei 110°C in die Schmelze von 840 g (4 mol) 2,4-Dichlor-3-fluornitrobenzol 488 g (8,4 mol) Kaliumfluorid und 40,0 g (0,07 mol) Dimethyl-di(ethoxypolyoxypropylmethylether)-ammoniumchlorid, 20,0 g (0,04 mol) Polyethylenglykoldimethylether 500 und 20,4 g (0,06 mol) Tetrabutylphosphoniumbromid eingetragen. Anschließend wurde mit 60 g (0,57 mol) Xylol azeotrop getrocknet und 21 Stunden bei einer Temperatur von 150°C gerührt. Entstandene Menge an 2,3,4-Trifluorbenzol: nach 5 Stunden: 13 GC-Flächen-%, nach 21 Stunden: 65 GC-Flächen-%.

### Vergleichsbeispiel: 2,4-Difluornitrobenzol

In einen 2-Liter Planschliffkolben mit Destillationsbrücke und Rührer wurden bei 140°C in die Schmelze von 1440 g (7,5 mol) 2,4-Dichlornitrobenzol 36 g (0,33 mol) Tetramethylammoniumchlorid, 72 g (0,15 mol) Polyethylenglykoldimethylether 500 und 1044 g (18 mol) Kaliumfluorid eingetragen. Anschließend wurden 108 g (1,02 mol) Xylol zugegeben und die Reaktionssuspension durch Anlegen eines Vakuums von 45 mbar und Erhitzen auf 160°C azeotrop getrocknet. Nachdem Xylol abdestilliert war, wurde die Destillationsbrücke gegen einen Rückflußkühler ausgetauscht, die Reaktionssuspension auf 200°C erhitzt und für 8 Stunden bei dieser Temperatur gerührt. Es wurden 177 g (15 % d.Th.) 2,4-Difluornitrobenzol und 1029 g 2,4-Dichlornitrobenzol und Nebenprodukte isoliert. Entstandene Menge an 2,4-Difluornitrobenzol nach GC-Analyse: nach 3 Stunden: 8 GC-Flächen-%, nach 8 Stunden: 17 GC-Flächen-%.

### Beispiel 9: 2,4-Difluornitrobenzol

### a) Startansatz

In einen 1,5 Liter Planschliffkolben mit Ankerrührer und Destillationsbrücke wurden bei 70°C in die Schmelze von 768 g (4,0 mol) 2,4-Dichlornitrobenzol 60 g (0,11 mol) Dimethyl-di(ethoxypolyoxypropylmethylether)-ammoniumchlorid, 10 g (0,02 mol) Polyethylenglykoldimethylether 500 und 465 g (8 mol) Kaliumfluorid portionsweise eingetragen. Anschließend wurde die Reaktionssuspension mit 30 g Xylol versetzt, auf 120°C erwärmt und unter vermindertem Druck azeotrop getrocknet. Nachfolgend wurde die Reaktionstemperatur auf 180°C erhöht und 12 Stunden bei dieser Temperatur gerührt. Anschließend wurde auf 70°C abgekühlt, mit 100 g Xylol versetzt und die entstandenen anorganischen Salze abfiltriert. Die Menge an isoliertem 2,4-Difluornitrobenzol betrug nach der Fraktionierung 370,5 g (58,1 % d. Th.), neben 220 g Zwischenprodukten und 2,4-Dichlornitrobenzol.

### b) Folgeansatz

Die 220 g Zwischenprodukte und 2,4-Dichlornitrobenzol aus Beispiel 9a) wurden mit 548,0 g (2,85 mol) frischem 2,4-Dichlornitrobenzol versetzt und die Reaktion analog dem Startansatz durchgeführt. Die Menge an isoliertem 2,4-Difluornitrobenzol betrug nach der Fraktionierung 420,2 g (92,7 % d. Th.) bezogen auf frisch eingesetztes 2,4-Dichlornitrobenzol, neben 160 g Zwischenprodukten und 2,4-Dichlornitrobenzol.

### Beispiel 10: 2,3,4-Trifluornitrobenzol

### a) Startansatz

In einen 1,5 Liter Planschliffkolben mit Ankerrührer und Destillationsbrücke wurden bei 70°C in die Schmelze von 850 g (4,0 mol) 2,4-Dichlor-3-fluornitrobenzol 54,9 g (0,1 mol) Dimethyl-di(ethoxypolyoxypropylmethylether)-ammoniumchlorid, 30 g (0,02 mol) Polyethylenglykoldimethylether 500 und 488 g (8,4 mol) Kaliumfluorid portionsweise eingetragen. Anschließend wurde die Reaktionssuspension mit 30 g Xylol versetzt, auf 110°C erwärmt und unter vermindertem Druck azeotrop getrocknet. Nachfolgend wurde die Reaktionstemperatur auf 150°C erhöht und 12 Stunden bei dieser Temperatur gerührt. Anschließend wurde auf 70°C abgekühlt, mit 100 g Xylol versetzt und die entstandenen anorganischen Salze abfiltriert. Die Menge an isoliertem 2,3,4-Trifluornitrobenzol betrug nach der Fraktionierung 439 g (62,0 % d. Th.), neben 145 g Zwischenprodukten und 2,4-Dichlor-3-fluornitrobenzol.

### b) Folgeansatz

Die 145 g Zwischenprodukte und 2,4-Dichlor-3-fluornitrobenzol aus Beispiel 10a) wurden mit 695,0 g (3,30 mol) frischem 2,4-Dichlor-3-fluornitrobenzol versetzt und die Reaktion analog dem Startansatz durchgeführt. Die Menge an isoliertem 2,3,4-Trifluornitrobenzol betrug nach der Fraktionierung 415,2 g (76,9 % d. Th.) bezogen auf frisch eingesetzte 2,4-Dichlor-3-fluornitrobenzol, neben 170 g Zwischenprodukten und 2,4-Dichlor-3-fluornitrobenzol.

## Patentansprüche

1. Verfahren zur Herstellung von mehrfach fluorierten Nitrobenzolen durch Umsetzung einer Verbindung der Formel (4) 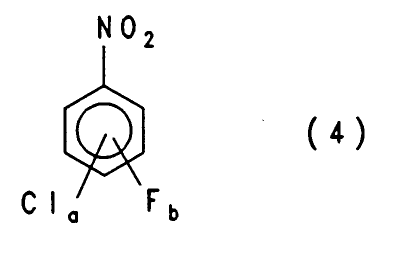 worin
a eine Zahl von 2 bis 4 und
b eine Zahl von 0 bis 2 ist,
mit einem Alkalimetallfluorid bei einer Reaktionstemperatur von 80 bis 220°C in Gegenwart eines Katalysators, dadurch gekennzeichnet, daß der Katalysator im wesentlichen aus
a) einer oder mehreren quartären Ammoniumverbindung(en) der Formel (1) 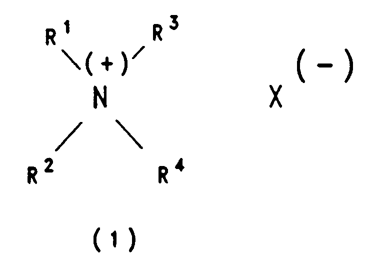 worin
R¹, R² und R³ gleich oder verschieden sind und
einen linearen oder verzweigten Alkoxypolyoxyalkyl-Rest der Formel -(CₘH₂ₘO)ₚR⁵ bedeuten, worin R⁵ für Wasserstoff oder einen linearen oder verzweigten Alkylrest mit 1 bis 16 Kohlenstoffatomen steht, m eine ganze Zahl von 1 bis 10 und p eine Zahl von 1 bis 15 bedeuten;
oder einen linearen oder verzweigten Alkylrest mit 1 bis 30 Kohlenstoffatomen; oder einen unsubstituierten Phenyl-oder Naphthylrest; oder einen substituierten Phenyl- oder Naphthylrest, wobei die Substituenten die Bedeutung Halogen, C₁-C₄-Alkyl, C₁-C₄-Alkoxy, Nitro oder Cyano haben;
R⁴ einen linearen oder verzweigten Alkoxypolyoxyalkyl-Rest der Formel -(CₘH₂ₘO)ₚR⁵ bedeuten; und
X^{⊖} ein anorganisches Anion ist;
oder aus einer Mischung der Komponente a) und
b) einem oder mehreren quartären Ammoniumsalz(en) oder Phosphoniumsalz(en) der Formel (2) 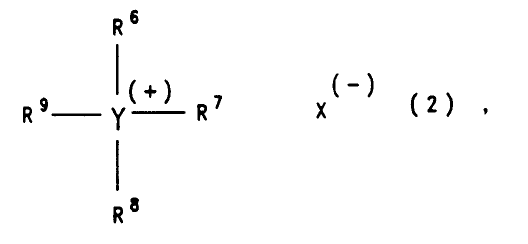 worin
R⁶, R⁷, R⁸ und R⁹ gleich oder verschieden sind und einen linearen oder verzweigten Alkylrest mit 1 bis 22 Kohlenstoffatomen; oder einen unsubstituierten oder substituierten Arylrest oder einen C₁-C₄-Alkyl-arylrest, wobei Aryl die Bedeutung Phenyl oder Naphthyl hat und die besagten Substituenten Halogen, C₁-C₄-Alkyl, C₁-C₄-Alkoxy, Nitro oder Cyano bedeuten; und
Y die Bedeutung N oder P hat;
oder aus einer Mischung der Komponente a) und
c) einem oder mehreren Polyether(n) der Formel (3)
R¹⁰-(O-CₓH₂ₓ)ᵣ-OR¹¹ (3),
worin
R¹⁰ und R¹¹ gleich oder verschieden sind und Wasserstoff oder einen linearen oder verzweigten Alkylrest mit 1 bis 16 Kohlenstoffatomen bedeuten,
x eine ganze Zahl von 2 bis 6 und
r eine Zahl von 0 bis 20 ist;
oder einem Kronenether;
oder aus einer Mischung der Komponenten a), b) und c) besteht.

2. Verfahren nach Anspruch 1, dadurch gekennzeichnet, daß der Katalysator im wesentlichen aus
a) einer oder mehreren quartären Ammoniumverbindung(en) der Formel (1) 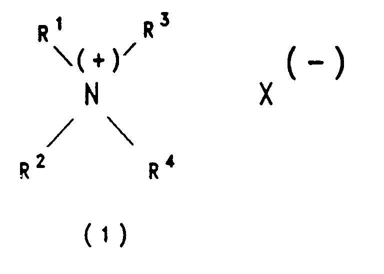 worin
R¹, R² und R³ gleich oder verschieden sind und einen linearen oder verzweigten Alkoxypolyoxyalkyl-Rest der Formel -(CₘH₂ₘO)ₚR⁵ bedeuten, worin R⁵ für Wasserstoff oder einen linearen oder verzweigten Alkylrest mit 1 bis 8 Kohlenstoffatomen steht, m eine ganze Zahl von 1 bis 5, und p eine Zahl von 2 bis 10 bedeuten;
oder einen linearen oder verzweigten Alkylrest mit 1 bis 18 Kohlenstoffatomen; oder einen unsubstituierten Phenyl-oder Naphthylrest; oder einen substituierten Phenyl- oder Naphthylrest, wobei die Substituenten die Bedeutung Halogen, C₁-C₄-Alkyl, C₁-C₄-Alkoxy, Nitro oder Cyano haben;
R⁴ einen linearen oder verzweigten Alkoxypolyoxyalkyl-Rest der Formel -(CₘH₂ₘO)ₚR⁵ bedeutet; und
X^{⊖} Fluorid, Chlorid, Bromid, SO₄²⁻/2 oder Hydrogensulfat ist;
oder aus einer Mischung der Komponente a) und b) einem oder mehreren quartären Ammoniumsalz(en) oder Phosphoniumsalz(en) der Formel (2) 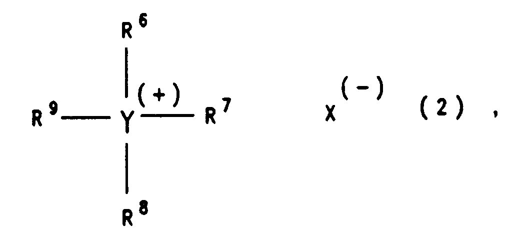 worin
R⁶, R⁷, R⁸ und R⁹ gleich oder verschieden sind und einen linearen oder verzweigten Alkylrest mit 1 bis 16 Kohlenstoffatomen; oder einen unsubstituierten oder substituierten Arylrest oder einen C₁-C₄-Alkyl-arylrest, wobei Aryl die Bedeutung Phenyl oder Naphthyl hat und die besagten Substituenten Halogen, C₁-C₄-Alkyl, C₁-C₄-Alkoxy, Nitro oder Cyano bedeuten; und
Y die Bedeutung N oder P hat;
oder aus einer Mischung der Komponente a) und
c) einem oder mehreren Polyether(n) der Formel (3)
R¹⁰-(O-CₓH₂ₓ)ᵣ-OR¹¹ (3),
worin
R¹⁰ und R¹¹ gleich oder verschieden sind und Wasserstoff oder einen linearen oder verzweigten Alkylrest mit 1 bis 8 Kohlenstoffatomen bedeuten,
x eine ganze Zahl von 2 bis 3 und
r eine Zahl von 4 bis 14 ist;
oder einem Kronenether;
oder aus einer Mischung der Komponenten a), b) und c) besteht.

3. Verfahren nach Anspruch 1 oder 2, dadurch gekennzeichnet, daß die Komponente a) mindestens 5 Gew.-%, vorzugsweise 20 bis 80 Gew.-%, des gesamten Katalysators ausmacht.

4. Verfahren nach mindestens einem der Ansprüche 1 bis 3, dadurch gekennzeichnet, daß in der Verbindung der Formel (1) ein oder zwei Alkoxypolyoxyalkyl-Reste enthalten sind.

5. Verfahren nach mindestens einem der Ansprüche 1 bis 4, dadurch gekennzeichnet, daß in der Verbindung der Formel (4) zwei oder drei, vorzugsweise zwei, Chloratome durch Fluoratome ausgetauscht werden.

6. Verfahren nach mindestens einem der Ansprüche 1 bis 5, dadurch gekennzeichnet, daß das Alkalimetallfluorid Kaliumfluorid, Rubidiumfluorid, Cäsiumfluorid oder eine Kombination dieser Fluoride, insbesondere Kaliumfluorid, ist.

7. Verfahren nach mindestens einem der Ansprüche 1 bis 6, dadurch gekennzeichnet, daß das molare Verhältnis von Katalysator zur Verbindung der Formel (4) gleich oder kleiner als 1:10, vorzugsweise 1:15 bis 1:50, ist.

8. Verfahren nach mindestens einem der Ansprüche 1 bis 7, dadurch gekennzeichnet, daß die Umsetzung in Abwesenheit eines Lösungsmittels durchgeführt wird.

9. Verfahren nach mindestens einem der Ansprüche 1 bis 8, dadurch gekennzeichnet, daß die Reaktionstemperatur bei 90 bis 180°C, insbesondere bei 120 bis 170°C, liegt.

10. Verfahren nach mindestens einem der Ansprüche 1 bis 9, dadurch gekennzeichnet, daß die Verbindung der Formel (4) 2,4-Dichlornitrobenzol, 2,4-Dichlor-3-fluornitrobenzol, 2,4-Dichlor-5-fluornitrobenzol, 2,4,5-Trichlornitrobenzol, 2,3,4-Trichlornitrobenzol, 2,3,4-Trichlor-5-fluornitrobenzol oder 2,4,5-Trichlor-3-fluornitrobenzol ist.

## Claims

1. A process for preparing multiply fluorinated nitrobenzenes by reaction of a compound of the formula (4) 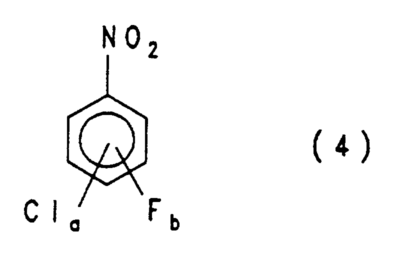 in which
a is a number from 2 to 4 and
b is a number from 0 to 2,
with an alkali metal fluoride at a reaction temperature of from 80 to 220°C in the presence of a catalyst, wherein the catalyst consists essentially of
a) one or more quaternary ammonium compound(s) of the formula (1) 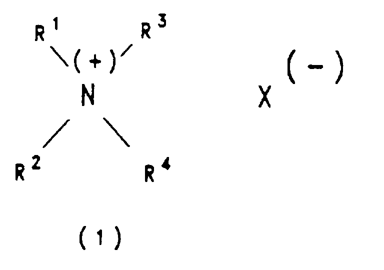 in which
R¹, R² and R³ are identical or different and are a linear or branched alkoxypolyoxyalkyl radical of the formula -(CₘH₂ₘO)ₚR⁵, in which R⁵ is hydrogen or a linear or branched alkyl radical having from 1 to 16 carbon atoms, m is an integer from 1 to 10 and p is a number from 1 to 15; or a linear or branched alkyl radical having from 1 to 30 carbon atoms; or an unsubstituted phenyl or naphthyl radical; or a substituted phenyl or naphthyl radical, with the substituents being halogen, C₁-C₄-alkyl, C₁-C₄-alkoxy, nitro or cyano;
R⁴ is a linear or branched alkoxypolyoxyalkyl radical of the formula -(CₘH₂ₘO)ₚR⁵; and
X^{⊖} is an inorganic anion;
or of a mixture of the component a) and
b) one or more quaternary ammonium salt(s) or phosphonium salt(s) of the formula (2) 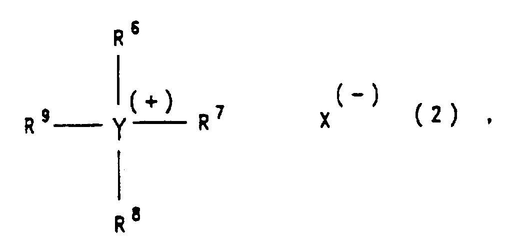 in which
R⁶, R⁷, R⁸ and R⁹ are identical or different and are a linear or branched alkyl radical having from 1 to 22 carbon atoms; or an unsubstituted or substituted aryl radical or a C₁-C₄-alkyl-aryl radical, with aryl being phenyl or naphthyl and said substituents being halogen, C₁-C₄-alkyl, C₁-C₄-alkoxy, nitro or cyano; and
Y is N or P;
or of a mixture of the component a) and
c) one or more polyether(s) of the formula (3)
R¹⁰-(O-CₓH₂ₓ)ᵣ-OR¹¹ (3),
in which
R¹⁰ and R¹¹ are identical or different and are hydrogen or a linear or branched alkyl radical having from 1 to 16 carbon atoms,
x is an integer from 2 to 6 and
r is a number from 0 to 20;
or a crown ether;
or of a mixture of the components a), b) and c).

2. The process as claimed in claim 1, wherein the catalyst consists essentially of
a) one or more quaternary ammonium compound(s) of the formula (1) 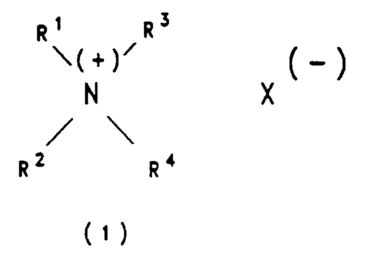 in which
R¹, R² and R³ are identical or different and are a linear or branched alkoxypolyoxyalkyl radical of the formula -(CₘH₂ₘO)ₚR⁵, in which R⁵ is hydrogen or a linear or branched alkyl radical having from 1 to 8 carbon atoms, m is an integer from 1 to 5, and p is a number from 2 to 10; or a linear or branched alkyl radical having from 1 to 18 carbon atoms; or an unsubstituted phenyl or naphthyl radical; or a substituted phenyl or naphthyl radical, with the substituents being halogen, C₁-C₄-alkyl, C₁-C₄-alkoxy, nitro or cyano;
R⁴ is a linear or branched alkoxypolyoxyalkyl radical of the formula -(CₘH₂ₘO)ₚR⁵; and
X^{⊖} is fluoride, chloride, bromide, SO₄²⁻/2 or hydrogen sulfate;
or of a mixture of the component a) and b) one or more quaternary ammonium salt(s) or phosphonium salt(s) of the formula (2) 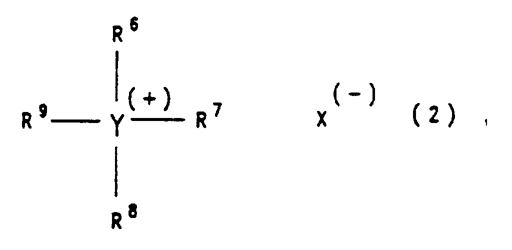 in which
R⁶, R⁷, R⁸ and R⁹ are identical or different and are a linear or branched alkyl radical having from 1 to 16 carbon atoms; or an unsubstituted or substituted aryl radical or a C₁-C₄-alkyl-aryl radical, with aryl being phenyl or naphthyl and said substituents being halogen, C₁-C₄-alkyl, C₁-C₄-alkoxy, nitro or cyano; and
Y is N or P;
or of a mixture of the component a) and
c) one or more polyether(s) of the formula (3)
R¹⁰-(O-CₓH₂ₓ)ᵣ-OR¹¹ (3),
in which
R¹⁰ and R¹¹ are identical or different and are hydrogen or a linear or branched alkyl radical having from 1 to 8 carbon atoms,
x is the integer 2 or 3 and
r is a number from 4 to 14;
or a crown ether;
or of a mixture of the components a), b) and c).

3. The process as claimed in claim 1 or 2, wherein the component a) makes up at least 5 % by weight, preferably from 20 to 80 % by weight, of the total catalyst.

4. The process as claimed in at least one of claims 1 to 3, wherein one or two alkoxypolyoxyalkyl radicals are present in the compound of the formula (1).

5. The process as claimed in at least one of claims 1 to 4, wherein two or three, preferably two, chlorine atoms in the compound of the formula (4) are replaced by fluorine atoms.

6. The process as claimed in at least one of claims 1 to 5, wherein the alkali metal fluoride is potassium fluoride, rubidium fluoride, cesium fluoride or a combination of these fluorides, in particular potassium fluoride.

7. The process as claimed in at least one of claims 1 to 6, wherein the molar ratio of catalyst to the compound of the formula (4) is equal to or less than 1:10, preferably from 1:15 to 1:50.

8. The process as claimed in at least one of claims 1 to 7, wherein the reaction is carried out in the absence of a solvent.

9. The process as claimed in at least one of claims 1 to 8, wherein the reaction temperature is from 90 to 180°C, in particular from 120 to 170°C.

10. The process as claimed in at least one of claims 1 to 9, wherein the compound of the formula (4) is 2,4-dichloronitrobenzene, 2,4-dichloro-3-fluoronitrobenzene, 2,4-dichloro-5-fluoronitrobenzene, 2,4,5-trichloronitrobenzene, 2,3,4-trichloronitrobenzene, 2,3,4-trichloro-5-fluoronitrobenzene or 2,4,5-trichloro-3-fluoronitrobenzene.

## Revendications

1. Procédé pour la préparation de nitrobenzènes fluorés plusieurs fois par réaction d'un composé de formule (4) 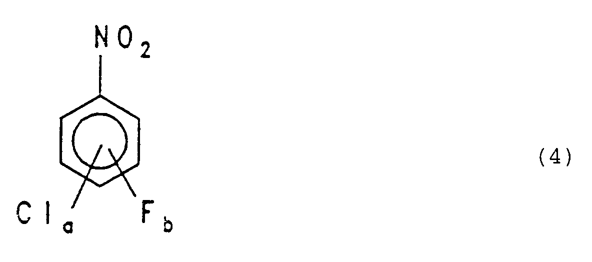 dans laquelle
a est un nombre de 2 à 4, et
b est un nombre de 0 à 2,
avec un fluorure de métal alcalin, à une température réactionnelle de 80 à 220 °C, en présence d'un catalyseur,
caractérisé en ce que le catalyseur est constitué essentiellement
a) d'un ou plusieurs composés d'ammonium quaternaire de formule (1) 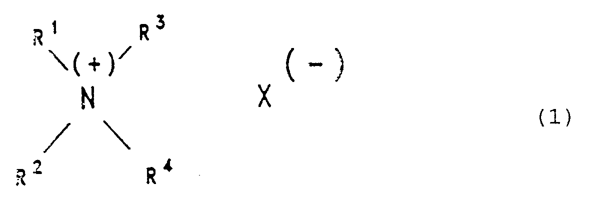 dans laquelle
R¹, R² et R³ sont identiques ou différents et représentent un reste alcoxypolyoxyalkyle linéaire ou ramifié de formule -(CₘH₂ₘO)ₚR⁵, dans laquelle R⁵ représente un atome d'hydrogène ou un reste alkyle linéaire ou ramifié avec 1 à 16 atomes de carbone, m est un nombre entier de 1 à 10 et p est un nombre de 1 à 15 ;
ou un reste alkyle linéaire ou ramifié avec 1 à 30 atomes de carbone ; ou un reste phényle ou naphtyle non substitué ; ou un reste phényle ou naphtyle substitué, les substituants ayant la signification d'atomes d'halogène, de restes alkyle en C₁-C₄, alcoxy en C₁-C₄, nitro ou cyano ;
R⁴ représente un reste alcoxypolyoxyalkyle linéaire ou ramifié de formule -(CₘH₂ₘO)ₚR⁵ ; et
X⁻ représente un anion inorganique ;
ou d'un mélange du composant a) et b) d'un ou plusieurs sels d'ammonium ou de phosphonium de formule (2) 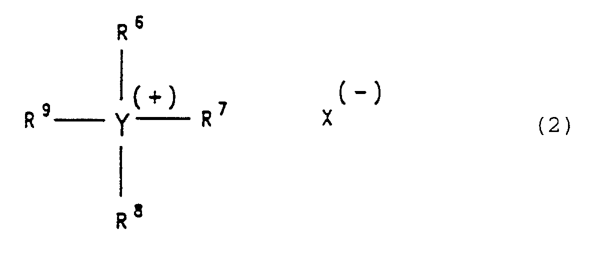 dans laquelle
R⁶, R⁷, R⁸ et R⁹ sont identiques ou différents et représentent un reste alkyle linéaire ou ramifié avec 1 à 22 atomes de carbone ; un reste (alkyl en C₁-C₄)aryle ou un reste aryle non substitué ou substitué, aryle ayant la signification de phényle ou de naphtyle et lesdits substituants représentant un atome d'halogène, des groupes alkyle en C₁-C₄, alcoxy en C₁-C₄, nitro ou cyano ; et
Y représente N ou P ;
ou d'un mélange du composant a) et
c) d'un ou plusieurs polyéthers de formule (3)
R¹⁰-(O-CₓH₂ₓ)ᵣ-OR¹¹ (3),
dans laquelle
R¹⁰ et R¹¹ sont identiques ou différents et représentent un atome d'hydrogène ou un reste alkyle linéaire ou ramifié avec 1 à 16 atomes de carbone,
x est un nombre entier de 2 à 6, et
r est un nombre de 0 à 20 ;
ou d'un éther-couronne ;
ou d'un mélange des composants a), b) et c).

2. Procédé selon la revendication 1, caractérisé en ce que le catalyseur est constitué essentiellement
a) d'un ou plusieurs composés d'ammonium quaternaire de formule (1) 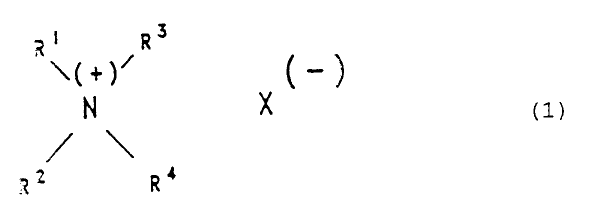 dans laquelle
R¹, R² et R³ sont identiques ou différents et représentent un reste alcoxypolyoxyalkyle linéaire ou ramifié de formule -(CₘH₂ₘO)ₚR⁵, dans laquelle R⁵ représente un atome d'hydrogène ou un reste alkyle linéaire ou ramifié avec 1 à 8 atomes de carbone, m est un nombre entier de 1 à 5 et p est un nombre de 2 à 10 ;
ou un reste alkyle linéaire ou ramifié 1 à 18 atomes de carbone ; ou un reste phényle ou naphtyle non substitué ; ou un reste phényle ou naphtyle substitué, les substituants ayant la signification d'atomes d'halogène, de restes alkyle en C₁-C₄, alcoxy en C₁-C₄, nitro ou cyano ;
R⁴ représente un reste alcoxypolyoxyalkyle linéaire ou ramifié de formule -(CₘH₂ₘO)ₚR⁵, et
X⁻ représente fluorure, chlorure, bromure, SO₄²⁻/2 ou bisulfate ;
ou d'un mélange du composant a) et
b) d'un ou plusieurs sels d'ammonium ou de phosphonium quaternaires de formule (2) 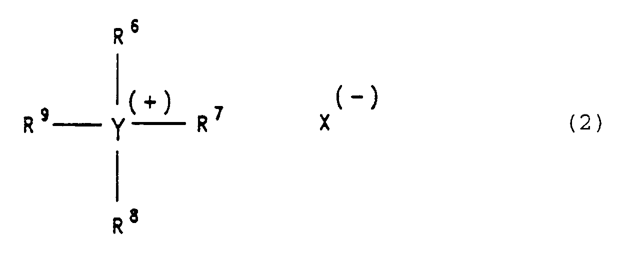 dans laquelle
R⁶, R⁷, R⁸ et R⁹ sont identiques ou différents et représentent un reste alkyle linéaire ou ramifié avec 1 à 16 atomes de carbone ; ou un reste aryle non substitué ou substitué ou un reste (alkyl en C₁-C₄)aryle, aryle ayant la signification de phényle ou de naphtyle, et lesdits substituants représentant un atome d'halogène, des groupes alkyle en C₁-C₄, alcoxy en C₁-C₄, nitro ou cyano ; et
Y représente N ou P ;
ou d'un mélange du composant a) et
c) d'un ou plusieurs polyéthers de formule (3)
R¹⁰-(O-CₓH₂ₓ)ᵣ-OR¹¹ (3),
dans laquelle
R¹⁰ et R¹¹ sont identiques ou différents et représentent un atome d'hydrogène ou un reste alkyle linéaire ou ramifié avec 1 à 8 atomes de carbone,
x est un nombre entier de 2 à 3 ; et
r est un nombre de 4 à 14 ;
ou d'un éther-couronne ;
ou d'un mélange de composants a), b) et c).

3. Procédé selon la revendication 1 ou 2, caractérisé en ce que le composant a) est contenu dans une quantité de 5 % en poids, de préférence de 20 à 80 % en poids par rapport à la totalité du catalyseur.

4. Procédé selon au moins l'une des revendications 1 à 3, caractérisé en ce que dans le composé de formule (1) sont contenus un ou deux restes d'alcoxypolyoxyalkyle.

5. Procédé selon au moins l'une des revendications 1 à 4, caractérisé en ce que dans le composé de formule (4), on échange deux ou trois, de préférence deux, atomes de chlore contre des atomes de fluor.

6. Procédé selon au moins l'une des revendications 1 à 5, caractérisé en ce que le fluorure de métal alcalin est le fluorure de potassium, le fluorure de rubidium, le fluorure de césium ou une combinaison de ces fluorures, plus particulièrement le fluorure de potassium.

7. Procédé selon au moins l'une des revendications 1 à 6, caractérisé en ce que le rapport molaire du catalyseur au composé de formule (4) est égal ou inférieur à 1:10, de préférence de 1:15 à 1:50.

8. Procédé selon au moins l'une des revendications 1 à 7, caractérisé en ce que l'on met en oeuvre la réaction en l'absence de solvant.

9. Procédé selon au moins l'une des revendications 1 à 8, caractérisé en ce que la température réactionnelle est de 90 à 180 °C, de préférence de 120 à 170 °C.

10. Procédé selon au moins l'une des revendications 1 à 9, caractérisé en ce que le composé de formule (4) est le 2,4-dichloronitrobenzène, le 2,4-dichloro-3-fluoronitrobenzène, le 2,4-dichloro-5-fluoronitro-benzène, le 2,4,5-trichloronitrobenzène, le 2,3,4-trichloronitrobenzène, le 2,3,4-trichloro-5-fluoronitro-benzène ou le 2,4,5-trichloro-3-fluoronitrobenzène.
